# EUROPEAN PATENT SPECIFICATION

(11) **EP 1 127 071 B1**
(45) Date of publication and mention of the grant of the patent: **04.08.2004**
(21) Application number: 99955835.6
(22) Date of filing: 03.11.1999
(51) Int. Cl.: C07K 14/60, C07K 5/06, A61K 38/25

(54) **COMPOUNDS WITH GROWTH HORMONE RELEASING PROPERTIES**
VERBINDUNGEN MIT WACHSTUMSHORMON-FREISETZENDER EIGENSCHAFT
COMPOSES PRESENTANT DES PROPRIETES DE LIBERATION D'HORMONE DE CROISSANCE

(30) Priority: 03.11.1998 DK 141498
(43) Date of publication of application: 29.08.2001
(73) Proprietor: Novo Nordisk A/S, 2880 Bagsvaerd (DK)
(72) Inventor: ANKERSEN, Michael, DK-2000 Frederiksberg (DK); RICHTER, Lutz, Stefan, deceased (DK)
(86) International application number: PCT/DK1999/000594
(87) International publication number: WO 2000/026252

(56) References cited:
- WO-A1-97/23508
- US-A- 5 977 178

## Description

### FIELD OF INVENTION

The present invention relates to novel compounds, compositions containing them, and their use for treating medical disorders resulting from a deficiency in growth hormone.

### BACKGROUND OF THE INVENTION

Growth hormone is a hormone which stimulates growth of all tissues capable of growing. In addition, growth hormone is known to have a number of effects on metabolic processes, e.g., stimulation of protein synthesis and free fatty acid mobilisation and to cause a switch in energy metabolism from carbohydrate to fatty acid metabolism. Deficiency in growth hormone can result in a number of severe medical disorders, e.g., dwarfism.

Growth hormone is released from the pituitary. The release is under tight control of a number of hormones and neurotransmitters either directly or indirectly. Growth hormone release can be stimulated by growth hormone releasing hormone (GHRH) and inhibited by somatostatin. In both cases the hormones are released from the hypothalamus but their action is mediated primarily via specific receptors located in the pituitary. Other compounds which stimulate the release of growth hormone from the pituitary have also been described. For example arginine, L-3,4-dihydroxyphenylalanine (L-Dopa), glucagon, vasopressin, PACAP (pituitary adenylyl cyclase activating peptide). muscarinic receptor agonists and a synthetic hexapeptide, GHRP (growth hormone releasing peptide) release endogenous growth hormone either by a direct effect on the pituitary or by affecting the release of GHRH and/or somatostatin from the hypothalamus.

In disorders or conditions where increased levels of growth hormone are desired, the protein nature of growth hormone makes anything but parenteral administration non-viable. Furthermore, other direct acting natural secretagogues, e.g., GHRH and PACAP, are longer polypeptides for which reason parenteral administration is preferred.

The use of certain compounds for increasing the levels of growth hormone in mammals has previously been proposed, e.g. in EP 18 072, EP 83 864, WO 8302272, WO 8907110, WO 8901711, WO 8910933, WO 8809780, WO 9118016, WO 9201711, WO 9304081, WO 9413696, WO 9517423, WO 9514666, WO 9615148, WO 9622997, WO 9635713, WO 9700894, WO 9722620, WO 9723508, WO 9740023, and WO 9810653.

The composition of growth hormone releasing compounds is important for their growth hormone releasing potency as well as their bioavailability. It is therefore an object of the present invention to provide novel compounds with growth hormone releasing properties. Moreover, it is an object to provide novel growth hormone releasing compounds (growth hormone secretagogues) which are specific and/or selective and have no or substantial no side-effects, such as e.g. release of LH, FSH, TSH, ACTH, vasopressin, oxytocin, cortisol and/or prolactin. It is also an object to provide compounds which have good oral bioavailability. A further object of the present Invention is to provide compounds with a relatively short plasma elimination half-life. A still further object of the present invention is to provide compounds which have a good oral biovailability together with a relatively short plasma elimination half-life.

### SUMMARY OF THE INVENTION

The present invention provides a novel compound or a pharmaceutically acceptable salt thereof, which acts directly on the pituitary cells under normal experimental conditions in vitro to release growth hormone therefrom.

The growth hormone releasing compound can be utilized in vitro as a unique research tool for understanding, inter alia, how growth hormone secretion is regulated at the pituitary level.

Moreover, the growth hormone releasing compound of the present invention can also be administered in vivo to increase endogenous growth hormone release.

### DESCRIPTION OF THE INVENTION

Accordingly, the present invention relates to the compound (2E)-5-Amino-5-methylhex-2-enoic acid N-methyl-N-[(1R)-1-(N-methyl-N-{(1R)-1-[N-methyl-N-(2-(2-pyridinyl)ethyl)carbamoyl]-2-phenylethyl}carbamoyl)-2-(2-naphthyl)ethyl]amide 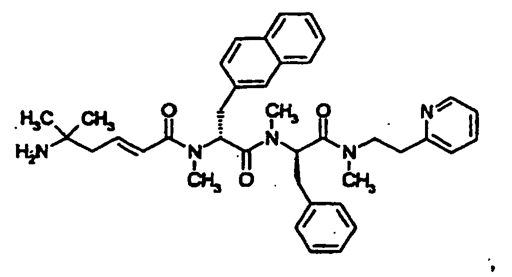 and pharmaceutically acceptable salts thereof.

The compound of the invention is believed to exhibit an improved resistance to proteolytic degradation by enzymes because it is non-natural, in particular because the natural amide bonds are replaced by non-natural amide bond mimetics. Such increased resistance to proteolytic degradation in comparison with known hormone releasing peptides is expected to improve bioavailability compared to that of the peptides suggested in the prior literature.

The compound of the present invention may optionally be in a pharmaceutically acceptable salt form, such as a pharmaceutically acceptable acid addition salt, including a salt prepared by reacting the compound with an inorganic or organic acid such as hydrochloric, hydrobromic, sulfuric, acetic, phosphoric, lactic, malic, maleic, mandelic, phthalic, citric, glutaric, gluconic, methanesulfonic, salicylic, succinic, tartaric, toluenesulfonic, trifluoracetic, sulfamic or fumaric acid and/or water.

The compound of the invention may be administered in pharmaceutically acceptable acid addition salt form. Such salt forms are believed to exhibit approximately the same order of activity as the free base form.

In another aspect, the present invention relates to a pharmaceutical composition comprising, as an active ingredient, the compound of the invention or a pharmaceutically acceptable salt thereof together with a pharmaceutically acceptable carrier or diluent.

Such pharmaceutical compositions may be prepared by conventional techniques, e.g. as described in Remington's Pharmaceutical Sciences, 1985 or in Remington: The Science and Practice of Pharmacy, 19th Edition (1995). The compositions may appear in conventional forms, for example capsules, tablets, aerosols, solutions, suspensions or topical applications.

The pharmaceutical carrier or diluent employed may be a conventional solid or liquid carrier. Examples of solid carriers are lactose, terra alba, sucrose, cydodextrin, talc, gelatin, agar, pectin, acacia, magnesium stearate, stearic acid or lower alkyl others of cellulose. Examples of liquid carriers are syrup, peanut oil, olive oil, phospholipids, fatty acids, fatty add amines, polyoxyethylene or water.

Similarly, the carrier or diluent may include any sustained release material known in the art, such as glyceryl monostearate or glyceryl distearate, alone or mixed with a wax.

If a solid carrier is used for oral administration, the preparation may be tabletted, placed in a hard gelatin capsule in powder or pellet form or it can be in the form of a troche or lozenge. The amount of solid carrier will vary widely but will usually be from about 25 mg to about 1 g. If a liquid carrier is used, the Preparation may be in the form of a syrup, emulsion, soft gelatin capsule or sterile injectable liquid such as an aqueous or non-aqueous liquid suspension or solution.

A typical tablet which may be prepared by convontional tabletting techniques may contain:

| Core: | |
|---|---|
| Active compound (as free compound or salt thereof) | 10mg |
| Colloidal silicon dioxide (Aerosil) | 1.5mg |
| Cellulose, microcryst. (Avicel) | 70mg |
| Modified cellulose gum (Ac-Di-Sol) | 7.5mg |
| Magnesium stearate | |
| | |

| Coating: | |
|---|---|
| HPMC approx. | 9mg |
| *Mywacett 9-40 T approx. | 0.9mg |

| | |
|---|---|
| *Acylated monoglyceride used as plasticizer for film coating, | |

For nasal administration, the preparation may contain the compound dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

It has been demonstrated that the compound of the invention possesses the ability to release endogenous growth hormone *in vivo*. The compound may therefore be used in the treatment of conditions which require increased plasma growth hormone levels, such as in growth hormone deficient humans, e.g. elderly patients, or in livestock. Furthermore, the compound of the invention has a high oral efficacy.

The pharmaceutical composition of the present invention may thus be a pharmaceutical composition for stimulating the release of growth hormone from the pituitary.

In a further aspect, the present invention relates to the use of the compound of the invention or a pharmaceutically acceptable salt thereof for the preparation of a medicament for stimulating the release of growth hormone from the pituitary of a mammal.

To those skilled in the art, it is well known that the current and potential uses of growth hormone in humans are varied and multitudinous. Thus, the compound of the invention or salt thereof can be administered for the purpose of stimulating release of growth hormone from the pituitary and would then have similar effects or uses as growth hormone itself. The compound or salt thereof is useful for: stimulation of growth hormone release in the elderly, prevention of catabolic side ejects of glucocorticoids, prevention and treatment of osteoporosis, treatment of chronic fatigue syndrom (CFS), treatment of acute fatigue syndrom and muscle loss following elective surgery, stimulation of the immune system, acceleration of wound healing, accelerating bone fracture repair, accelerating repair in complicated fractures, e.g. in distraction osteogenesis, treatment of wasting secondary to fractures, treatment of growth retardation, treating growth retardation resulting from renal failure or insufficiency, treatment of cardiomyopathy, treatment of wasting in connection with chronic liver disease, treatment of thrombocytopenia, treatment of growth retardation in connection with Crohn's disease, treatment of short bowel syndrome, treatment of wasting in connection with chronic obstructive pulmonary disease (COPD), treatment of complications associated with transplantation, treatment of physiological short stature including growth hormone deficient children and short stature associated with chronic illness, treatment of obesity and growth retardation associated with obesity, treatment of anorexia, treating growth retardation associated with the Prader-Will syndrome and Turner's syndrome; increasing the growth rate of a patient having partial growth hormone insensitive syndrome, accelerating the recovery and reducing hospitalization of bum patients; treatment of intrauterine growth retardation, skeletal dysplasia, hypercortisolism and Cushing's syndrome; induction of pulsatile growth hormone release; replacement of growth hormone in stressed patients, treatment of osteochondrodysplasias, Noonan's syndrome, schizophrenia, depressions, Alzheimer's disease, delayed wound heating and psychosocial deprivation, treatment of catabolism in connection with pulmonary dysfunction and ventilator dependency; treatment of cardiac failure or rotated vascular dysfunction, treatment of impaired cardiac function, treatment or prevention of myocardial infarction, lowering blood pressure, protection against ventricular dysfunction or prevention of reperfusion events; treatment of adults in chronic dialysis; attenuation of protein catabolic responses after major surgery, reducing cachexia and protein loss due to chronic illness such as cancer or AIDS; treatment of hyperinsulinemia including nesidioblastosis, adjuvant treatment for ovulation induction; stimulation of thymic development and prevention of the age-related decline of thymic function, treatment of immunosuppressed patients; treatment of sarcopenia, treatment of wasting in connection with AIDS; improvement in muscle strength, mobility, maintenance of skin thickness, metabolic homeostasis and renal homeostasis in the frail elderly, stimulation of osteoblasts, bone remodelling and cartilage growth; regulation of food intake; stimulation of the immune system in companion animals and treatment of disorder of aging in companion animals, promoting growth in livestock and stimulation of wool growth in sheep, increasing milk production in livestock, treatment of metabolic syndrom (syndrome X), treatment of insulin resistance, inducing NIDDM, in mammals, e.g. humans, treatment of insulin resistance in the heart, improvement of sleep quality and correction of the relative hyposomatotropism of senescence due to high increase in REM sleep and a decrease in REM latency, treatment of hypothermia, treatment of frailty associated with aging, treatment of congestive heart failure, treatment of hip fractures, treatment of immune deficiency in individuals with a depressed T4/T8 cell ratio, treatment of muscular atrophy, treatment of musculoskeletal impairment in the elderly, enhancing the activity of protein kinase B (PKB), improvement of the overall pulmonary function, treatment of sleep disorders, treatment of growth retardation in connection with asthma, treatment of growth retardation in connection with juvenile rheumatic arthritis, and treatment of growth retardation in connection with systic fibrosis.

For the above indications the dosage of the compound of the invention or pharmaceutically acceptable salt thereof will vary depending on the mode of administration and on the therapy desired. However, generally dosage levels between 0.0001 and 100 mg/kg body weight daily are administered to patents and animals to obtain effective release of endogenous growth hormone. Morever, the compound of the invention is believed to have no or substantially no side-effects, when administered in the above dosage levels, such side-effects being e.g. release of LH, FSH, TSH, ACTH, vasopressin, oxytocin, cortisol and/or prolactin. Usually, dosage forms suitable for oral, nasal, pulmonal or transdermal administration comprise from about 0.0001 mg to about 100 mg, preferably from about 0.001 mg to about 50 mg of the compound of the invention admixed with a pharmaceutically acceptable carrier or diluent.

The dosage of the compound according to this invention is suitably 0.01-500 mg/day, e.g. from about 5 to about 50 mg, such as about 10 mg per dose, when administered to patients, e.g. humans, as a drug.

Optionally, the pharmaceutical composition of the invention may comprise the compound of the invention or a pharmaceutically acceptable salt thereof, combined with one or more compounds exhibiting a different activity, e.g., an antibiotic or other pharmacologically active material.

The route of administration may be any route which effectively transports the active compound to the appropriate or desired site of action, such as oral, nasal, pulmonary, transdermal or parenteral, the oral route being preferred.

Apart from the pharmaceutical use of the compound of the invention or salts thereof, they may be useful in vitro tools for investigating the regulation of growth hormone release.

The compound of the invention or salts thereof may also be useful in vivo tools for evaluating the growth hormone releasing capability of the pituitary. For example, serum samples taken before and after administration to humans can be assayed for growth hormone. Comparison of the growth hormone content in each serum sample would directly determine the ability of the patients pituitary to release growth hormone.

The compound of the invention or salts thereof may be administered to commercially important animals to increase their rate and extent of growth, and to increase milk or wool production.

A further use of the growth hormone secretagogue compound of the invention or salt thereof is in combination with other secretagogues such as GHRP (2 or 6), GHRH and its analogues, growth hormone and its analogues or somatomedins including IGF-1 and IGF-2.

### Pharmacological Methods

The compound of the invention and salts thereof may be evaluated in vitro for their efficacy and potency to release growth hormone in rat pituitary primary cultures, and such evaluation may be performed as described below.

The isolation of rat pituitary cells is a modification of the procedure of O. Sartor et al., Endocrinology 116, 1985, pp. 952-957. Male albino Sprague-Dawley rats (250 +/- 25 grams) were purchased from Møllegaard, Lille Skensved, Denmark. The rats were housed in group cages (four animals/cage) and placed in rooms with 12 hour light cycle. The room temperature varied from 19-24°C and the humidity from 30-60%.

The rats were decapitated and the pituitaries dissected. The neurointermediate lobes were removed and the remaining tissue was immediately placed in icecold isolation buffer (Gey's medium (Gibco 041-04030) supplemented with 0.25% D-glucose, 2% non-essential amino acids (Gibco 043-01140) and 1% bovine serum albumin (BSA) (Sigma A-4503)). The tissue was cut into small pieces and transferred to isolation buffer supplemented with 3.8 mg/ml of trypsin (Worthington #3707 TRL-3) and 330 mg/ml of DNase (Sigma D-4527). This mixture was incubated at 70 rotations/min for 35 min at 37°C in a 95/5% atmosphere of O₂/CO₂. The tissue was then washed three times in the above buffer. Using a standard pasteur pipette, the tissue was then aspirated into single cells. After dispersion, cells were filtered through a nylon filter (160 mm) to remove undigested tissue. The cell suspension was washed 3 times with isolation buffer supplemented with trypsin inhibitor (0.75 mg/ml, Worthington #2829) and finally resuspended in culture medium; DMEM (Gibco 041-01965) supplemented with 25 mM HEPES (Sigma H-3375), 4 mM glutamine (Gibco 043-05030H), 0.075% sodium bicarbonate (Sigma S-8875), 0.1% non-essential amino acid, 2.5% fetal calf serum (FCS, Gibco 011-06290), 3% horse serum (Gibco 034-06050), 10% fresh rat serum, 1 nM T₃ (Sigma T-2752) and 40 mg/l dexamethasone (Sigma D-4902) pH 7.3, to a density of 2 x 10⁵ cells/ml. The cells were seeded into microtiter plates (Nunc, Denmark), 200 ml/well, and cultured for 3 days at 37°C and 8% CO₂.

### Compound testing

After culturing, the cells were washed twice with stimulation buffer (Hanks Balanced Salt Solution (Gibco 041-04020) supplemented with 1% BSA (Sigma A-4503), 0.25% D-glucose (Sigma G-5250) and 25 mM HEPES (Sigma H-3375) pH 7.3) and preincubated for 1 hour at 37°C. The buffer was exchanged with 90 ml stimulation buffer (37°C). Ten ml test compound solution was added and the plates were incubated for 15 min at 37°C and 5% CO₂-. The medium was decanted and analyzed for GH content in an rGH SPA test system.

The compound was tested in doses in the range from 10 pM to 100 mM. A dose-response relation was constructed using the Hill equation (Fig P, Biosoft). The efficacy (maximal GH released, Eₘₐₓ) was expressed in % of the Eₘₐₓ of GHRP-6. The potency (EC₅₀) was determined as the concentration inducing half maximal stimulation of the GH release.

### EXAMPLES:

The preparation of the compound of the invention and preparations containing it is described and illustrated in the following.

The structures of the various compounds prepared in this connection are confirmed by either elemental analysis (MA) nuclear magnetic resonance (NMR) or mass spectrometry (MS). NMR shifts (δ) are given in parts per million (ppm) and only selected peaks are given, mp is melting point and is given in °C Column chromatography was carried out using the technique described by W.C. Still et al, J. Org. Chem.1978. 43, 2923-2925 on silica gel 60. Compounds used as starting materials are either known compounds or compounds which can readily be prepared by methods known per se.

### HPLC-Analysis:

### Method A1.

The RP-analysis was performed using UV detections at 214, 254, 276, and 301 nm on a 218TP54 4.6 mm x 250 mm 5m C-18 silica column (The Separations Group. Hesperia). which was eluted at 1 mL/min at 42°C. The column was equilibrated with 5% acetonitrile in a buffer consisting of 0.1 M ammonium sulfate, which was adjusted to pH 2.5 with 4M sulfuric acid, after injection the sample was eluted by a gradient of 5% to 60% acetonitrile in the same buffer during 50 min.

### Method B1.

The RP-analysis was performed using UV detections at 214, 254, 276, and 301 nm on a 218TP54 4.6 mm x 250 mm 5m C-18 silica column (The Separations Group, Hesperia), which was eluted at 1 mL/min at 42°C. The column was equilibrated with 5% (acetonitrile + 0.1 % TFA) in an aqueous solution of TFA in water (0.1%). After injection the sample was eluted by a gradient of 5% to 60% (acetonitrile + 0.1 % TFA) in the same aqueous buffer during 50 min.

### Abbreviations:

- TLC:: thin layer chromatography
- DMSO:: dimethylsulfoxide
- min:: minutes
- h:: hours
- Boc:: tert butyloxycarbonyl
- DMF:: dimethylformamide
- THF:: tetrahydrofuran
- EDAC:: N-ethyl-N'-dimethylaminopropylcarbodiimide hydrochloride
- HOAt:: 1-hydroxy-7-azabenzotriazole
- DIEA:: diisopropylethylamine
- TFA:: trifluoroacetic acid

### Building blocks:

N-methylated aminoacids used in the following were prepared as in Can. J. Chem. 1977, 55, 906.

### 3-Hydroxy-1,1-dimethylpropylcarbamic acid tert-butyl ester:

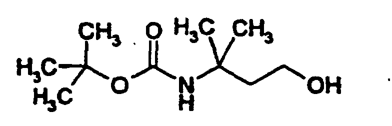

At 0°C, ethyl chloroformate (1.10 mL, 11.5 mmol) was given dropwise to a solution of 3-tert-butoxycarbonylamino-3-methylbutanoic add (2.50 g, 11.5 mmol) and triethylamine (1.92 mL, 13.8 mmol) in tetrahydrofuran (10 mL). The solution was stirred for 40 min at 0 °C. The formed precipitate was filtered off and washed with tetrahydrofuran (20 mL). The liquid was immediately cooled to 0 °C. A 2M solution of lithium boronhydride In tetrahydrofuran (14.4 mL, 28.8 mmol) was added dropwise. The solution was stirred at 0 °C for 2 h, and then warmed to room temperature, over a period of 4 h. It was cooled to 0 °C. Methanol (5 mL) was added carefully. 1N Hydrochloric acid (100 mL) was added. The solution was extracted with ethyl acetate (2 x 100 mL, 3 x 50 mL). The combined organic layers were washed with saturated sodium hydrogen carbonate solution (100 mL) and dried over magnesium sulfate. The solvent was removed in vacuo. The crude product was chromatographed on silica (110 g) with ethyl acetate/heptane 1:2 to give 1.84 g of 3-hydroxy-1,1-dimethylpropylcarbamic acid tert-butyl ester.
¹H-NMR (CDCl₃): **δ** 1.33 (s, 6 H); 1.44 (s, 9H); 1.88 (t, 2 H); 1.94 (br, 1H); 3.75 (q, 2 H); 4.98 (br, 1 H).

### 3-(tert-Butoxycarbonylamino)-3-methylbutanal:

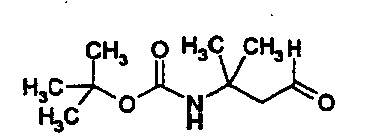

DMSO (1.22 mL, 17.2 mmol) was added to a solution of oxalyl chloride (1.1 mL, 12.9 mmol) at -78°C in dichloromethane (15 mL). The mixture was stirred for 15 min at -78 °C. A solution of 3-hydroxy-1,1-dimethylpropylcarbamic add tert-butyl ester (1.75 g, 8.6 mmol) in dichloromethane (10 mL) was added dropwise over a period of 15 min. The solution was stirred at -78 °C for another 15 min. Triethylamine (6.0 mL, 43 mmol) was added. The solution was stirred at -78 °C for 5 min and then warmed to room temperature. The solution was diluted with dichloromethane (100 mL) and extracted with 1N hydrochloric acid (100 mL). The aqueous phase was extracted with dichloromethane (50mL). The combined organic layers were washed with saturated sodium hydrogen carbonate solution (100 mL) and dried over magnesium sulfate. The solvent was removed in vacuo. The crude product was purified by column chromatography on silica (140 g) with ethyl acetate/heptane (1:3) to give 1.10 g of 3-(tert-butoxycarbonylamino)-3-methylbutanal.
¹H-NMR (CDCl₃): δ 1.39 (s, 6 H); 1.45 (s, 9 H); 2.85 (d, 2 H); 4.73 (br. 1H); 9.80 (t,1H).

### Ethyl (2E)-5-(tert-Butoxycarbonylamino)-5-methylhex-2-enoate:

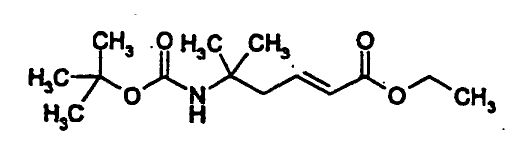

Triethylphoshonoacetate (1.96 ml, 9.8 mmol) was dissolved in tetrahydrofuran (30 ml). Potassium tert-butoxide (1.10 g, 9.8 mmol) was added. The solution was stirred for 40 min at room temperature. A solution of 3-(tert-butoxycarbonylamino)-3-methylbutanal (1.10 g, 5.5 mmol) in Tetrahydrofuran (6 ml) was added. The solution was stirred at room temperature, for 75 min. It was diluted with ethyl acetate (100 ml) and 1N hydrochloric add (100 ml). The phases were separated. The aqueous phase was extracted with ethyl acetate (2 x 50 ml). The combined organic phases were washed with saturated sodium hydrogen carbonate solution (60 ml) and dried over magnesium sulfate. The solvent was removed in vacuo. The crude product was purified by column chromatography on silica (90 g) with ethyl acetate/hepatane (1:4) to give 1.27 g of ethyl (2E)-5-(tert-butoxycarbonylamino)-5-methylthex-2-enoate.
¹H-NMR (CDCl₃): δ 1.30 (s, 6 H); 1.30 (t, 3 H); 1.46 (s, 9 H); 2.62 (d, 2 H); 4.27 (q, 2 H); 4.42 (br, 1H); 5.88 (d, 1 H); 6.94 (td, 1 H).

### (2E)-5-(bert-Butoxycarbonylamino)-5-methylhex-2-enoic acid:

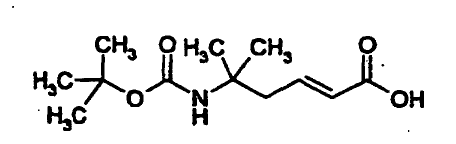

Ethyl (2E)-5-(tert-butoxycarbonylamino)-5-methylhex-2-enoate (1.233 g, 4.54 mmol) was dissolved in dioxane (20 ml). Lithium hydroxide (0.120 g. 5.00 mmol) was added as a solid. Water (10 ml) was added, until a clear solution was reached. The solution was stirred 16 h at room temperature. The solution was diluted with water (70 mn and was extracted with tert-butyl methyl ether (2 x 100 ml). The aqueous phase was acidified with 1N sodium hydrogensulfate solution (pH = 1) and was extracted with tert-butylmethylether (3 x 70 ml). The organic phases were combined and dried over magnesium sutfate. The solvent was removed in vacuo to give 1.05 g of (2E)-5-(tert-butoxycarbonylamino)-5-methylhex-2-enoic acid. The crude product was used for further syntheses.
¹H-NMR (DMSO d₆): δ 1.15 (s, 6 H); 1.35 (s, 9 H): 2.53 (d, 2 H); 5.75 (d, 1H); 6.57 (br, 1 H); 6.75 (td, 1H); 12.15 (s,1H).

### Example 1

### (2E)-5-Amino-5-methylhex-2-enoic acid N-methyl-N-[(1R)-1-(N-methyl-N-{(1R)-1-[N-methyl-N-(2-(2-pyridinyl)ethyl)carbamoyl]-2-phenylethyl}carbamoyl)-2-(2-naphthyl)ethyl]amide.

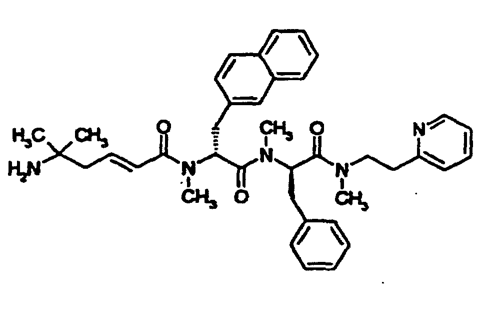

### Step 1.

### N-Methyl-N-{(1R)-1-[N-methyl-N-(2-(2-pyridyl)ethyl)carbamoyl]-2-phenylethyl}carbamic acid tert-butyl ester.

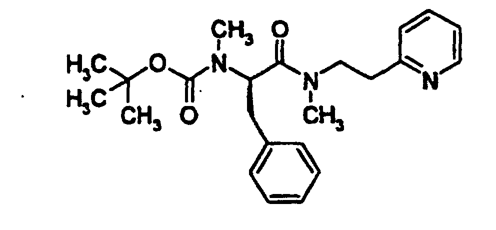

(2R)-2-(N-(tert-Butoxycarbonyl)-N-methylamino)-3-phenylpropionic acid (11.2 g, 40 mmol). 1-hydroxy-7-azabenzotriazole (5.4 g, 40 mmol) and N-(3-dimethylaminopropyl)-N'-ethylcarbodiimide hydrochloride (7.7 g, 40 mmol) were dissolved in dichloromethane (100 mL). The reaction mixture was stirred for 15 min. Ethyldiisopropylamine (6.85 mL, 40 mmol) and 2-(2-methylaminoethyl)pyridine (5.54 mL, 40 mmol) were added. The reaction mixture was stirred for 16 h. The reaction mixture was washed with sat, aqueous sodium hydrogen carbonate solution (3 x 150 mL). The organic layer was dried over magnesium sulfate. The solvent was removed in vacuo. The crude product was purified by flash chromatography on silica (400 g), using ethyl acetate as eluent, to give 10.9 g of N-methyl-N-{(1R)-1-{N-methyl-N-(2-(2-pyridyl)ethyl)carbamoyl}-2-phenylethyl)carbamic acid tert-butyl ester.
¹H-NMR (CDCl₃): δ 1.12, 1.20, 1.30 and 1.35 (all s, together 9 H); 2.75-3.20 (m, together 10 H); 3.50-4.00 (m, together 2 H), 4.95, 5.23 and 5.39 (m,t and t, together 1 H); 7.00-7.30 (m, together 7 H); 7.55 (m, 1H); 8.51 (br m, 1H)

### Step 2.

### (2R)-N-Methyl-2-(methylamino)-3-phenyl-N-(2-(2-pyridyl)ethyl)propionamide.

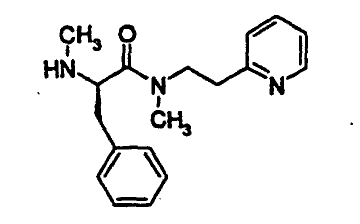

N-Methyl-N-{(1R)-1-{N-methyl-N-(2-(2-pyridyl)ethyl)carbamoyl}-2-phenylethyl}carbamic acid tert-butyl ester was dissolved in dichloromethane (75 mL). Trifluoroacetic acid (75 mL. 0.978 mol) was added to the stirred solution. The reaction mixture was stirred for 40 min. The solvent was removed in vacuo. The product was dissolved in dichloromethane (30 mL) and sat. aqueous sodium hydrogen carbonate solution (20 mL). The reaction mixture was neutralised with solid sodium hydrogen carbonate. Dichloromethane (100 mL) was added and the aqueous phase was extracted with dichloromethane (2 x 150 mL). The combined organic layers were dried over magnesium sulfate. The solvent was removed in vacuo, to give 7.9 g of (2R)-N-Methyl-2-(methylamino)-3-phenyl-N-(2-(2-pyridyl)ethyl)propionamide.
¹H-NMR (CDCl₃): δ 2.10 and 2.27 (both s, together 3 H); 2.51 and 2.87 (both s, together 3 H); 2.60-3.20 (m, together 4 H); 3.50-3.75 (m, together 3 H); 6.97-7.3 (m, together 7 H); 7.57 (m, 1 H); 8.50 (dd, 1 H)

### Step 3.

### N-Methyl-N-[(1R)1-(N-methyl-N-{(1R)-1-[N-methyl-N-(2-(pyridinyl)carbamoyl]-2-phenylethyl}carbamoyl)-2-(2-naphthyl)ethyl]carbamic acid tert-butyl ester.

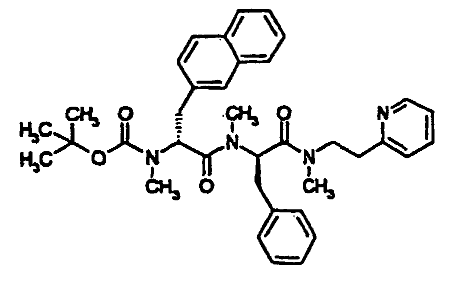

(2R)-2-(N-(tert-butoxycarbonyl)-N-methylamino)-3-(2-naphthyl) propionic acid (11.2 g, 34 mmol), 1-hydroxy-7-azabenzotriazole (5.2 g, 38 mmol) and N-(3-dimethylaminopropyl)-N'-ethylcarbodiimide hydrochloride (6.9 g, 36 mmol) were dissolved in dichloromethane (100 mL). The reaction mixture was stirred for 15 min. Ethyldiisopropylamine (7.0 mL, 41 mmol) was added. (2R)-N-Methyl-2-(methylamino)-3-phenyl-N-(2-(2-pyridyl)ethyl)propionamide (7.9 g, 27 mmol) dissolved in dichloromethane (20 mL) was added. The reaction mixture was stirred for 16 h. The reaction mixture was added to aminomethylresin (17.3 g, 13.5 mmol). The reaction mixture was stirred for 6 h. The reaction mixture was filtrated. The organic layer was washed with sat. aqueous sodium hydrogen carbonate solution (2 x 150 mL). The organic layer was dried over magnesium sulfate. The solvent was removed in vacuo. The crude product was purified by flash chromatography on silica (400 g), using ethyl acetate as eluent, to give 14.9 g N-Methyl-N-[(1R)1-(N-methyl-N-{(1R)-1-[N-methyl-N-(2-(pyridinyl)ethyl)carbamoyl]-2-phenylethyl}carbamoyl)-2-(2-naphthyl)ethyl]carbamic acid tert-butyl ester.
¹H-NMR (CDCl₃): δ 1.07, 1.15, 1.33 and 1.40 (all s, together 9 H); 1.80-3.75 (m, together 17 H); 5.00, 5.35, 5.59, 5.70 and 5.85 (m, together 2 H); 6.85-7.80 (m, together 15 H); 8.40-8.57 (m. together 1H).

### Step 4.

### (2R)-N-Methyl-2-(methylamino)-N-{(1R)-1-[N-methyl-N-(2-(pyridin-2-yl)ethyl)carbamoyl]-2-phenylethyl}-3-(2-naphthyl)propionamide.

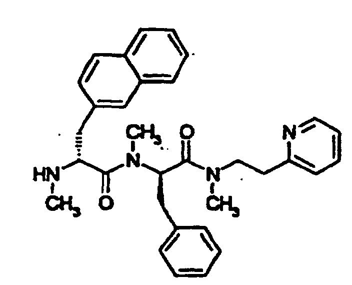

N-Methyl-N-[(1R)1-(N-methyl-N-{(1R)-1-[N-methyl-N-(2-(pyridinyl)ethyl)carbamoyl]-2-phenylethyl}carbamoyl)-2-(2-naphthyl)ethyl]carbamic acid tert-butyl ester (14.9 g, 24.5 mmol) was dissolved in dichloromethane (75 mL). Trifluoroacetic acid (75 mL, 0.978 mol) was added to the stirred solution. The reaction mixture was stirred for 40 min. The solvent was removed in vacuo. The product was dissolved in dichloromethane (50 mL) and sat. aqueous sodium hydrogen carbonate solution (40 mL). The mixture was neutralised with solid sodium hydrogen carbonate. Dichloromethane (100 mL) was added and the aqueous phase was extracted with dichloromethane (2 x 150 mL). The combined organic layers were dried over magnesium sulfate. The solvent was removed in vacuo, to give 11.9 g of (2R)-N-Methyl-2-(methylamino)-N-[(1R)-1-[N-methyl-N-(2-(pyridin-2-yl)ethyl)carbamoyl]-2-phenylethyl]-3-(2-naphthyl)propionamide.
¹H-NMR (CDCl₃): δ 1.77, 1.87 and 1.97 (all s, together 4 H); 2.60 (s, 3 H); 2.70-3.20 (m, together 9 H); 3.52, 3.70 and 4.10 (t, m and m, together 2 H); 5.72-5.89 (m, 1 H); 6.90-7.80 (m, together 15 H); 8.50 (dd, 1 H).

### Step 5.

### ((3E)-1,1-Dimethyl-4-{N-methyl-N-[(1R)1-(N-methyl-N-{(1R)-1-[N-methyl-N-(2-(pyridin-2-yl)ethyl)carbamoyl]-2-phenylethyl}carbamoyl)-2-(2-naphthyl)ethyl]carbamoyl}but-3-enyl)carbamic acid tert-butyl ester.

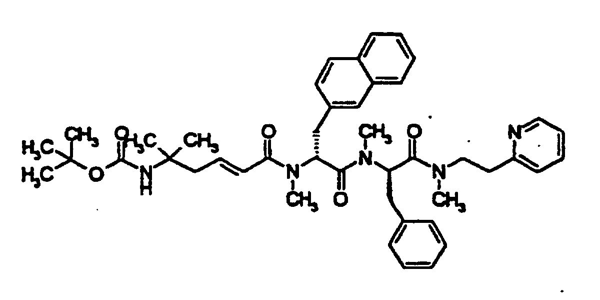

(2E)-5-(tert-Butoxycarbonylamino)-5-methylhex-2-enoic acid (2.87 g, 11.8 mmol), 1-hydroxy-7-azabenzotriazole (1.6 g, 11.8 mmol) and N-(3-dimethylaminopropyl)-N'-ethylcarbodiimide hydrochloride (2.26 g, 11.8 mmol) were dissolved in dichloromethane (30 mL). The reaction mixture was stirred for 15 min. Ethyldiisopropylamine (2.0 mL, 11.8 mmol) was added. (2R)-N-Methyl-2-(methylamino)-N-{(1R)-1-[N-methyl-N-(2-(pyridin-2-yl)ethyl)carbamoyl]-2-phenylethyl}-3-(2-naphthyl)propionamide (6.0 g, 11.8 mmol) dissolved in dichloromethane (10 mL) was added. The reaction mixture was stirred for 16 h. The reaction mixture was washed with sat aqueous sodium hydrogen carbonate solution (100 mL). The aqueous phase was extracted with dichloromethane (100 mL). The combined organic layers were dried over magnesium sulfate. The solvent was removed in vacuo. The crude product was purified by flash chromatography on silica (400 g), using ethyl acetate as eluent, to give 5.28 g of ((3E)-1,1-Dimethyl-4-{N-methyl-N-[(1R)1-{N-methyl-N-{(1R)-1-[N-methyl-N-(2-(pyridin-2-yl)ethyl)carbamoyl]-2-phenylethyl}carbamoyl)-2-(2-naphthyl)ethyl]carbamoyl}but-3-enyl)carbamic acid tert-butyl ester.
¹H-NMR (CDCl₃): δ 1.17-1.29 (m, 6 H); 1.40 (s, 9 H); 2-40-3.10 (m, together 16 H); 3.37-3.75 (m, together 3 H); 4.40 (br m, 1 H); 5.55-5.37 (m, together 2 H); 6.03-6.19 (m, 1 H); 6.70-7.80 (m, together 15 H); 8.36-8.55 (m, together 1 H).

### Step 6.

((3E)-1,1-Dimethyl-4-{N-methyl-N-[(1R)1-(N-methyl-N-[(1R)-1-[N-methyl-N-(2-(pyridin-2-yl)ethyl)carbamoyl]-2-phenylethyl}carbamoyl)-2-(2-naphthyl)ethyl]carbamoyl}but-3-enyl)carbamic acid tert-butyl ester (5.28 g, 7.2 mmol) was dissolved in dichloromethane (50 mL). The solution was cooled to -10 °C. Trifluoroacetic acid (50 mL) was added to the stirred solution. The reaction mixture was stirred for 45 min at -10 °C. The reaction mixture was added to a solution of ice, sodium hydrogen carbonate and water. The reaction mixture was neutralised with sodium hydrogen carbonate. Dichloromethane (400 mL) was added. The aqueous phase was extracted with dichloromethane (300 mL). The combined organic layers were dried over magnesium sulfate. The solvent was removed in vacuo. The crude product was purified by flash chromatography on silica (40 g), using 15% of a solution, 7% ammonium in ethanole, in dichloromethane as eluent, to give 3.2 g of the title compound.
¹H-NMR (CDCl₃): δ 0.80-120 (m, together 6 H); 2.05-3.10 (m, together 17 H); 3.25-3.70 (m, together 3 H); 5.62, 5.70 and 5.85 (m,m and m, together 2 H); 6.03-6.17 (m, 1 H); 6.80-7.78 (m, together 15 H); 8.38-8.58 (m, 1H)
- MS:: 634.2
- HPLC:: 28.243 min (A1)
28.963 min (B1)

For biological testing, the title compound was transformed into its acetate salt by lyophilization from 0.5 M acetic acid (100mL)

### Comparison example

The compound of example 1 was compared with the compound of example 408 disclosed in WO 9723508. The results are presented below.

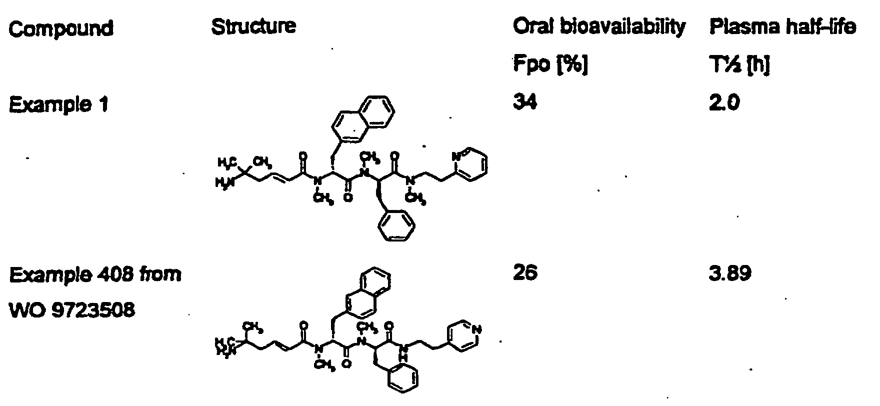

As can be seen, the compound of example 1 shows a better oral bioavailability and a shorter plasma half-life compared with the compound of example 408 from WO 972350B.

The pharmakokinetic data were obtained by the following procedure:

The pharmacokinetics of the test compounds were Investigated in fasted Beagle dogs.

Intravenous and oral administration of the test compound, in 5% glucose solution, was separated by a one-week washout.

Blood samples were collected immediately before drug administration (time zero) and than 0.08, 0.25, 0.50, 0.75, 1.0, 1.5. 2.0, 3.0, 4.0, 5.0, and 6.0 hours after administration.

The plasma samples were stored frozen (<-18°C) pending analysis.

An HPLC method with solid phase extraction and W detection was used for the quantification of the compound in plasma.

The pharmacokinetic parameters for the compounds were calculated by non-compartmental methods using the PC based pharmacokinetic software WinNonlin, version 1.1 (Scientific Consulting Inc., Apex, NC, USA).

## Claims

1. The compound:
(2E)-5-Amino-5-methylhex-2-enoic acid N-methyl-N-[(1R)-1-(N-methyl-N-{(1R)-1-[N-methyl-N-(2-(2-pyridinyl)ethyl)carbamoyl]-2-phenylethyl}carbamoyl)-2-(2-naphthyl)ethyl]amide 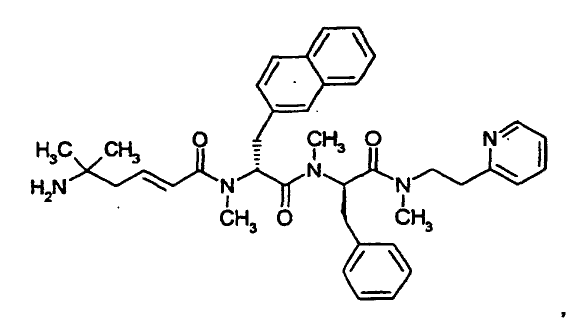 or a pharmaceutical acceptable salt thereof.

2. A pharmaceutical composition comprising, as an active ingredient, the compound or pharmaceutically acceptable salt thereof according to claim 1 together with a pharmaceutically acceptable carrier or diluent.

3. Use of the compound or pharmaceutically acceptable salt thereof according to claim 1 for the preparation of a medicament for stimulating the release of growth hormone from the pituitary of a mammal.

## Patentansprüche

1. Verbindung (2E)-5-Amino-5-methylhex-2-ensäure-N-methyl-N-[(1R)-1-(N-methyl-N-{(1R)-1-[N-methyl-N-(2-(2-pyridinyl)ethyl)carbamoyl]-2-phenylethyl}carbamoyl)-2-(2-naphthyl)ethyl]amid 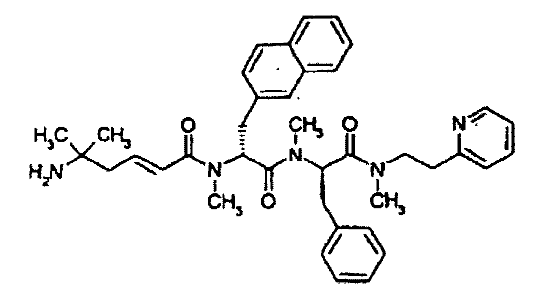 oder ein pharmazeutisch verträgliches Salz davon.

2. Arzneimittel, umfassend als Wirkstoff die Verbindung oder ein pharmazeutisch verträgliches Salz davon nach Anspruch 1 zusammen mit einem pharmazeutisch verträglichen Träger.

3. Verwendung der Verbindung oder eines pharmazeutisch verträglichen Salzes davon nach Anspruch 1 zur Herstellung eines Medikaments zum Stimulieren der Freisetzung von Wachstumshormon von der Hypophyse eines Säugers.

## Revendications

1. Le composé N-méthyl-N-[(1R)-1-(N-méthyl-N-{(1R)-1-[N-méthyl-N-(2-(2-pyridinyl)éthyl)carbamoyl]-2-phényléthyl}carbamoyl)-2-(2-naphtyl)éthyl]amide de l'acide (2E)-5-amino-5-méthylhex-2-énoïque 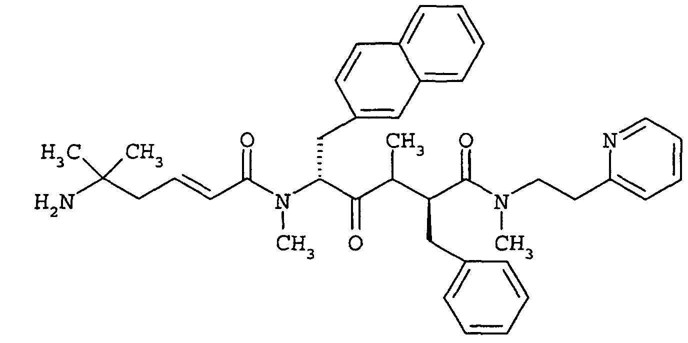 ou l'un de ses sels acceptables d'un point de vue pharmaceutique.

2. Composition pharmaceutique comprenant en tant que principe actif le composé ou l'un de ses sels acceptables d'un point de vue pharmaceutique selon la revendication 1, en même temps qu'un excipient ou un diluant acceptable d'un point de vue pharmaceutique.

3. Utilisation du composé ou d'un de ses sels acceptables d'un point de vue pharmaceutique selon la revendication 1 pour préparer un médicament destiné à stimuler la libération de l'hormone de croissance à partir de l'hypophyse d'un mammifère.
